# EUROPEAN PATENT APPLICATION

(11) **EP 1 550 452 A1**
(43) Date of publication of application: **06.07.2005**
(21) Application number: 03029901.0
(22) Date of filing: 29.12.2003
(51) Int. Cl.: A61K 35/78, A61P 9/10, A61P 9/14, A61K 31/616, A61K 31/609, A61K 31/557

(54) **Composition comprising an aqueous extract of red vine leaves and a antthrombotic agent for the treatment of chronic venous insufficiencies**

(71) Applicant: Boehringer Ingelheim International GmbH, 55216 Ingelheim am Rhein (DE)
(72) Inventor: Okada, Minoru, Chiba 270-1323 (JP); Horie, Toshiaki, Chiba 287-0205 (JP); Takahashi, Koichi, Chiba 286-0202 (JP); Masuda, Kenji, Saitama 340-0052 (JP)

(57) **Abstract**

This invention relates to a new composition containing the effective dosage of an aqueous extract of red vine leaves **(1)** and an antithrombotic agent **(2)** for preventing or alleviating the discomfort associated with mild-to-moderate chronic venous insufficiency of the legs. The compositions according to this invention may also contain pharmaceutically or dietetically acceptable additives.

## Description

### BACK-GROUND OF THE INVENTION

### 1. Technical Field

The invention relates to compositions comprising an effective dose of an aqueous extract of red vine leaves and an antithrombotic agent for preventing or alleviating mild-to-moderate chronic venous insufficiency of the legs. The composition according to this invention also includes acceptable pharmaceutical or dietetic additives. In addition, the compositions according to this inventions decrease or prevent subjective symptoms such as lassitude (listlessness), heavy legs, tired legs, sensation of tension, and pain associated with swelling of calves and ankles due to disorder of leg venous flow.

### 2. Related Art

Presently, there are millions of people around the world who suffer from mild-to-moderate chronic venous insufficiency of the legs. This common condition is characterized by an inadequacy of the venous circulation to return blood from the legs to the heart. The lack of adequate venous return results in venous stasis and an increased pressure within the venous circulation, promoting the development of oedema and tissular water retention. Chronic venous insufficiency (CVI) is a functional disorder caused by persistent inadequacy of the venous return and is characterized clinically by oedema, skin changes and subjective complaints such as tired, heavy legs, pain or tingling sensations, which are typically amplified by standing upright and by high ambient temperatures. This dysfunction may be a source of major distress with a significant negative impact on the patient's overall well-being and quality of life.

Early stages (grade I) are characterized by coronal phlebectasia paraplantaris, subfascial congestion and oedema; grade II CVI is associated with low-grade skin changes, eczema and lipodermatosclerosis. If untreated, grades I and II often progress to an advanced stage characterized by recurrent venous leg ulcers (grade III). The stress caused by the symptoms, even when relatively mild initially, and the risk of later complications call for appropriate supportive and preventive measures to be initiated in the early stages of CVI.

Although some patients, even at early stages, might require surgery (sclerotherapy and variceal surgery), the use of compression stockings with or without additional physiotherapy is the most common treatment approach. The effect of compression is merely mechanical, i.e. this approach does not affect or correct the related biological dysfunction (capillary fragility in particular). Furthermore, the treatment with compression stockings often lacks compliance because of cosmetic concerns and the overall inconvenience of the compressive stockings, in the summer in particular. Therefore there is an urgent need for alternative approaches that are effective, well-tolerated and more convenient.

This extract of red vine leaves contains flavon (ol) -glycosides, -glucuronides and flavonoids, with quercetin-3-O-beta-D-glucuronide and isoquercitrin (quercetin-3-O-beta-glucoside) as its main active ingredients. The range of their pharmacological actions has not yet been fully elucidated, but in-vitro studies indicate that they have antioxidant and anti-inflammatory properties and that they inhibit platelet aggregation and hyaluronidase and reduce oedema, possibly by reducing capillary permeability. Preclinical in-vivo experiments demonstrated anti-inflammatory and capillary wall thickening effects.

Dietary supplements including an aqueous extract of red vine leaves are disclosed to prevent and reduce the discomfort relating to mild-to-moderate chronic venous insufficiency of the legs in WO 01/28363. However, there are no hints to compositions comprising an aqueous extract of red vine leaves and other active ingredients such as antithrombotic agents given by WO 01/28363.

### SHORT DESCRIPTION OF THE INVENTION

Surprisingly, potentiation of anti-inflammatory action and inhibitory action on oedema, indices of pharmacological activities of an aqueous extract of red vine leaves, is found by combination of an antithrombotic agent with an aqueous extract of red vine leaves comparing the action itself. Moreover, composing mild antithrombotic agents resulted in safe compositions whose efficacy is potentiated for preventing and alleviating discomfort relating to mild-to-moderate chronic venous insufficiency of the legs with minimum or no adverse reactions. The new compositions comprising an antithrombotic agent and an aqueous extract of red vine leaves potentiate the efficacy of prevention or relaxation for mild-to-moderate chronic venous insufficiency of the legs.

Therefore, this invention relates to new compositions that comprise an effective dose of an aqueous extract of red vine leaves and an antithrombotic agent as pharmacological active substances and their efficacies are potentiated for preventing and relaxing mild-to-moderate chronic venous insufficiency of the legs.

### Objective of the present invention

A primary objective of this invention provides more effective internal compositions for preventing and alleviating the discomfort associated with mild-to-moderate chronic venous insufficiency of the legs.

A further objective of this invention provides more effective internal compositions including herb components and a antithrombotic agent. The herb components were manufactured pursuant to a controlled process that preserves the herbal effectiveness of the ingredients for preventing and/or alleviating the discomfort associated with mild-to-moderate chronic venous insufficiency of the legs.

Another objective of this invention provides more effective internal compositions including herb components and an antithrombotic agent with minimum or no adverse event for safety of internal consumption that prevent and/or alleviate the discomfort associated with mild-to-moderate chronic venous insufficiency of the legs.

The other objective of this invention provides more effective internal pharmaceutical compositions and foods for preventing and/or alleviating the discomfort associated with mild-to-moderate chronic venous insufficiency of the legs.

### DETAILLED DESCRIPTION OF THE INVENTION

This invention relates to internal compositions for preventing or alleviating the discomfort associated with mild-to-moderate chronic venous insufficiency of the legs including an effective dose of an aqueous extract of red vine leaves and a antithrombotic agent.

The internal composition of this invention consists of herbal ingredients derived from an aqueous extraction (*Extractum vitis viniferae e folium spissum et siccum*) of red vine leaves (*folia vitis viniferae*) and a antithrombotic agent.

The primary active ingredient of the internal composition is the aqueous extract of red vine leaves (*foliae vitis viniferae L.*).
The term "aqueous extract of red vine leaves" in this invention means the aqueous or solid aqueous extract of red vine leaves manufactured pursuant to a controlled process that preserves the herbal effectiveness of the ingredients. The term "dried extract of red vine leaves" in this invention means dried pure extract of the above aqueous extract of red vine leaves. The term "red vine leaves extract" in this invention means solid extracts added with silicon dioxide in the range of 1 to 10 (wt/wt)% (described as %) and glucose syrup (as dried material) in the range of 5 to 25 % to the above dried extract of red vine leaves (solid pure extracts) in the range of 70 to 90 %.

Red vine leaves as starting material for the aqueous extract of red vine leaves in this invention is also known as "dyer" which are leaves of *vitis vinifera* LINNE with blackish-blue pericarp and a red pulp. Concentration of each polyphenol compound in red vine leaves and its composition are affected by various ecophysiological factors around. It is preferred that dried leaves of red vine containing at least 4 % of total polyphenols and 0.2 % of anthocyans are used as starting material in this invention. Red vine leaves
characterized like those are harvested at a point of time where the content of flavonoids has reached an optimum i.e. around the harvesting time of the grapes. Moreover, less than 15 cm length and less than 12 cm width of red vine leaves are preferable. The leaves are carefully dried and crushed. For extraction the leaves are cut to pieces of preferably 5 to 10 mm. To achieve a high content of flavonoids the extraction is done using purified water at elevated temperature, preferably at a temperature in the range of 60 to 80 °C, over a time of at least 6 up to 10 hours. The preferred method is that of an exhaustive percolation.
The so-called fluid extract obtained in the process of the extraction may be directly used in the preparation of liquid dosage forms. In order to get a more concentrated extract, at least a part of the solvent is removed by use of a suitable evaporator preferably.
The thick extract is sterilized under heated-compressed condition, preferably at a temperature from 120 to 150°C for 1 up to 30 seconds, more preferably at a temperature from 140 to 145°C for 2 up to 5 seconds. The thick extract obtained in this step may again be directly used in the manufacturing of liquid dosage forms.
For the preparation of solid dosage forms the thick extract is dried, for instance by use of a vacuum drying oven or a vacuum drying conveyer. Carriers or excipients may be added during drying to facilitate further processing of the extract.

The ratio of carriers or excipients in the range of 10 to 30 % and dried extract of red vine leaves (as pure extract) in the range of 70 to 90 % in red vine leaves extract is preferable. Such carriers or excipients exemplify one or more than 2 kinds among silicon dioxide, maltodextrine, glucose syrup, cellulose and others. Silicon dioxide and glucose syrup are preferably used in this invention. The ratio of silicon dioxide in the range of 1 to 10 %, glucose syrup (as dried) in the range of 5 to 25 % and dried extract of red vine leaves (as pure extract) in the range of 70 to 90 % in red vine leaves extract is preferable. The ratio of silicon dioxide 2-5 %, glucose syrup (as dried) 10-20 % and dried extract of red vine leaves (as pure extract) 75-85 % in red vine leaves extract is more preferable.

The aqueous extract of red vine leaves used in this invention by pure extract conversion of an aqueous extract of red vine leaves contains total flavonoids (quercetin-3-O-beta-D-glucuronide) preferably in the range of 0.625 to 25 %, more preferably in the range of 1.25 to 12.5 %, specially in the range of 2.5 to 10 %. This total flavonoid (quercetin-3-O-beta-D-glucuronide) content in red vine leaves extract (for example, a case in which dried extract of red vine leaves (as pure extract) 80 %) is preferable from 0.5 to 20 %, more preferable from 1 to 10 %, special from 2 to 8 %.

To prevent and/or alleviate the discomfort of mild-to-moderate chronic venous insufficiency of the legs, the daily dosage of the aqueous extract of red vine leaves for an adult in equivalent quantity of dried extract of red vine leaves (pure extract) is usually from 64 to 800mg, preferably from 240 to 640 mg, more preferably from 280 to 600 mg and further more preferably 360 mg. The daily dosage of the aqueous extract of red vine leaves for an adult in equivalent quantity of red vine leaves extract is usually from 80 to 1000mg, preferably from 300 to 800 mg, more preferably 350 to 750 mg and further more preferably 450 mg.

The compositions according to this invention include antithrombotic agents as second active ingredients in addition to above aqueous extract of red vine leaves.

Antithrombotic agents used in this invention are not limited and determined if the agents contain antithrombotic action, however, for safety of this agent with minimum or no adverse event, antithrombotic agents with mild effects used in non-prescription drug and health food field for many years are preferable. In addition, types and dosage of antithrombotic agents change depending on whether this internal composition is pharmaceutical products or health foods.

Examples of such antithrombotic agents are aspirin, aspirin dialuminate, ethenzamide, salicylamide, sodium salicylate, docosahexaenoic acid (DHA), eicosapentaenoic acid (EPA), crude drugs and herbs having antithrombotic action, and etc. These antithrombotic agents can be used in one or mixed with more than two kinds.

Examples of such crude drugs and herbs having the antithrombotic action are cnidium rhizome (*Cnidii rhizoma*), cinnamon ramus (*Cinnamomum ramulus*), danshen root (*Salviae* *radix*), turmeric (*Curcuma longa*), safflower (*Carthamus flos*), umbelliferae (*Angelica keisukei*), notoginseng root (*Notoginseng radix*), American ginseng root (*Panacis quinquefolii radix*), Reishi (*Ganoderma lucidum*), melilot herb (*Melilotus herba*), propolis, bacillus natto, nattokinase, chitin, chitosan. In addition, these crude drug and herb having antithrombotic action can be dried powder, extract, and fluidextract, etc.

Combination amount of antithrombotic agent used in this invention components changes depending on types of antithrombotic agents and categorization as pharmaceutical products or foods, but a daily dose for an adult is between 1 to 10,000 mg.

Specifically, a combination amount of aspirin, aspirin · dialuminate, ethenzamide, salicylamide and sodium salicylate for an adult is usually between 1 and 4000 mg, preferably between 2 and 3500 mg, more preferably between 5 and 3000 mg. A daily combination amount of docosahexaenoic acid and icosapentaenoic acid (IPA) is usually between 1 to 2,000 mg for an adult, preferably between 3 to 1,500 mg, more preferably between 5 to 1,000 mg.

A daily combination amount of herb and crude having antithrombotic action is usually between 1 to 10,000 mg as crude drug substance for an adult, preferably between 2 to 8,000 mg, more preferably 3 to 6,000 mg.

The compositions according to this invention may be administered parentherally, preferably orally in divided doses, most preferably given once a day in the morning, especially before breakfast. Dose adjustment of the active ingredients may reflect age, body weight, and manifesting symptoms. In addition to active ingredients mentioned above, the internal compositions in this invention may also include other active ingredients.

The oral dosage form described in this invention can be used in various types of oral forms as tablets, granules, fine granules, powders, capsules, caplets, soft capsules, pills, oral solutions, syrups, dry syrups, chewable tablets, troches, effervescent tablets, drops, suspension, oral fast-dispersing tablets, etc. Any of these formulations may be prepared using regular methods, and, in addition to the aforementioned components, any excipients in common use may be used upon preparation of these formulations, if necessary. In addition, preparations formed into microparticles such as microcapsules, nanocapsules, microspheres, nanospheres, and included in the aforementioned formulations. For example, the active ingredients, i.e. an aqueous extract of red vine leaves and antithrombotic agents, can be various types of drug forms as separate granules, multi-layer granules, multi-layer tablets or dry coated tablets, tablets of separated granules, microcapsules, etc. Coating preparations such as sugarcoated tablets, film coating tablets, coating granule, can be used as well as chewable tablets, oral fast dispersing tablets, matrix tablets, matrix granules, effervescent tablets, dusting powder, solid solutions, etc. These methods can also be combined. Moreover, the properties of the inventive internal composition such as stability, release, continuance, disintegration, distinglation, dissolution, concealment of taste, improvement in usage etc. can be regulated by the addition of additives known in the art.

These oral dosage form described in this invention may be prepared using regular methods by adding generally available pharmaceutical additives and food additives such as excipients, binders, disintegrators, lubricants, coating agents, sugar coating agents, plasticizers, antifoaming agents, polish, foaming agents, antistatic agents, desiccant, surfactant, solubilizer, buffer agents, resolvents, solubilizing agents, solvents, diluents, stabilizers, emulsifying agents, suspension, suspending agents, dispersing agents, isotonizing agents, adsorbents, reducing agents, antioxidant, wetting agents, wet modifier, filler, extender, adhesives, viscous agent, softeners, pH modifiers, antiseptics, preservatives, sweetening agents, corrigent, refrigerative agents, flavoring agents, perfume, fragrance, and coloring matters to the active compounds. Examples of such additives are described in Japanese Pharmaceutical Excipients Directory 2000 (edited by Japan Pharmaceutical Excipients Council, issued by Yakuji Nippo, Ltd.) and The Japan's Specifications and Standards for Food Additives (issued by Japan Food Additives Association).

The compositions according to this invention can be provided as pharmaceutical products or foods. The compositions described in this invention are explained by the following practical examples. However, the scope of this invention is not limited to these practical examples.

### Examples

### Example 1

### Capsules

The following ingredients were prepared as granules through regular methods, and capsule-filled to give an amount of 300 mg per one capsules.

| | |
|---|---|
| Red vine leaves extract | 450 g |
| Nattokinase | 300 g |
| Corn starch | 105 g |
| Light anhydrous silicic acid | 9 g |
| Talc | 27 g |
| Magnesium stearate | 9 g |
| (Red vine leaves extract = dried aqueous extract of red vine leaves (pure extract): silicon dioxide: glucose syrup (as dried glucose)) = 80: 3: 17 wt/wt%) | |

### Example 2

### Granules

The following ingredients were prepared as granules through a regular method to prepare mixed particles, and packed to give an amount of 2000 mg per one pack for granules.

| | |
|---|---|
| Red vine leaves extract | 225 g |
| Garlic extract | 100 g |
| Melilot herb extract | 50 g |
| Calcium carboxymethylcellulose | 80 g |
| Mannitol | 360 g |
| Corn starch | 165 g |
| Tartaric acid | 8 g |
| Aspartame | 8 g |
| Acesulfame potassium | 3 g |
| Fragrant materials | 1 g |
| (Red vine leaves extract = dried aqueous extract of red vine leaves (pure extract): silicon dioxide: glucose syrup (as dried glucose)) = 80: 3: 17 wt/wt%) | |

### Example 3

### Powder

The following ingredients were homogeneously mixed. The resulted mixed particles were divided into portions of 850 mg to prepare powder compositions.

| | |
|---|---|
| Red vine leaves extract | 450 g |
| Cnidium rhizome extract | 100 g |
| (Cnidium rhizome | 800g) |
| Notoginseng root extract | 50 g |
| (Notoginseng root | 500g) |
| Corn starch | 124 g |
| Lactose | 108 g |
| Light anhydrous silicic acid | 10 g |
| Magnesium stearate | 8 g |
| (Red vine leaves extract = dried aqueous extract of red vine leaves (pure extract): silicon dioxide: glucose syrup (as dried glucose)) = 80: 3: 17 wt/wt%) | |

### Example 4

### Tablet

The following ingredients were homogeneously mixed. The resulted mixed particles were compressed with a mold to prepare tablets at 300 mg each.

| | |
|---|---|
| Red vine leaves extract | 450 g |
| Aspirin dialuminate | 81 g |
| Potassium L-aspartate | 20 g |
| Lactose | 100 g |
| Microcrystalline cellulose | 255 g |
| Light anhydrous silicic acid | 7 g |
| Magnesium stearate | 5 g |
| Talc | 2 g |
| (Red vine leaves extract = dried aqueous extract of red vine leaves (pure extract): silicon dioxide: glucose syrup (as dried glucose)) = 80: 3: 17 wt/wt%) | |

## Claims

1. A composition for the prevention and/or alleviation of mild-to-moderate chronic venous insufficiency (CVI) of the legs comprising an aqueous extract of red vine leaves (1) and an antithrombotic agent (2) as pharmacologically active substances.

2. A composition according to claim 1 containing an aqueous extract of red vine leaves (1), which is obtained by extraction from dried red vine leaves containing at least 4 % of total polyphenols and at least 0.2 % of anthocyans using purified water.

3. A composition according to claim 1 or 2, which contains from 50 to 1000 mg, preferably 64 to 800 mg, in particular about 360 mg or 450 mg of dried aqueous extract of red vine leaves (1).

4. A composition according to any one of claims 1 to 3, which contains from 0.625 to 25 % by weight, preferably from 2.5 and 10 % by weight of flavonoids in the dried aqueous extract of red vine leaves (1).

5. A composition according to any one of claims 1 to 4, which contains dried aqueous extract of red vine leaves (1) and an excipient.

6. A composition according to any one of claims 1 to 5, which contains from 70 and 90 % by weight, preferably 75 to 85 % by weight of dried aqueous extract of red vine leaves (1) and from 10 to 30 % by weight, preferably 10 to 20 % by weight of an excipient.

7. A composition according to any one of claims 1 to 6, which contains 2 to 5 % by weight of silicon dioxide and 10 to 20 % by weight of glucose syrup.

8. A composition according to any one of claims 1 to 7, wherein the antithrombotic agent (2) is selected from the group consisting of aspirin, aspirin dialuminate, ethenzamide, salicylamide, sodium salicylate, docosahexaenoic acid, eicosapentaenoic acid, crude drugs and herbs having antithrombotic action or a mixture thereof.

9. A composition according to any one of claims 1 and 8, which contains 1 to 10,000 mg of one or more antithrombotic agents (2).

10. A composition according to any one of claims 1 and 9, wherein the weight ratio between the dried aqueous extract of red vine leaves (1) to the antithrombotic agent (2) is from 1 to 500 to 1,000 to 1.

11. A composition according to any one of claims 1 and 10, which is suitable for parenteral, preferably oral administration.

12. Use of a composition as claimed in any one of claims 1 to 11 for the preparation of a pharmaceutical product or a health food for the prevention and/or alleviation of mild-to-moderate chronic venous insufficiency (CVI) of the legs.
